# EUROPEAN PATENT APPLICATION

(11) **EP 2 450 081 A2**
(43) Date of publication of application: **09.05.2012**
(21) Application number: 11181506.4
(22) Date of filing: 15.09.2011
(51) Int. Cl.: A61M 39/06, A61M 39/04

(54) **Sealing perforable closing device and medical apparatus associable to said device**

(30) Priority: 12.10.2010 IT BO20100609
(71) Applicant: Tipromed S.r.l., 42041 Brescello (RE) (IT)
(72) Inventor: Minari, Emanuelle, I-42041 Brescello (RE) (IT)
(74) Representative: Negrini, Elena

(57) **Abstract**

A sealing perforable closing device is associable to a medical apparatus provided with a housing for said device (1) and engageable by a needle along a piercing direction. Said device (1) comprises a body means (2) comprising an absorbent material (6) of liquids. In an operative condition of extraction of the needle, possible liquids contacting the absorbent material are absorbed and they cause the increasing of volume of the material the same and of the body means (2) that expanding itself seals the perforation crossing the body means (2).

## Description

The present invention pertains to the field of the disposable medical products and it refers to a sealing perforable closing device assigned to provide the sealing also after the needle, piercing it, is removed.

Sealing perforable closing device of known kind are used in medical instruments as, for instance, the closed venous catheter systems, the injections tips for fluid administration devices or for dialysis, sample test tubes and closures for bottles containing injectable liquids, for making a pneumatic and/or hydraulic sealing between a needle and a tubular body means or container and for sealing the latter when the device is not holed by the needle and when the needle is removed.

The perforable closing device associated to the bottles containing injectable liquids consist of septum or closures made of elastomer material blocked into the neck hole of the bottle by means of fasteners or caps made of plastic or of metal. Said known devices can be pierced by a needle at a tip point for using the liquid; the hole made by the needle closes spontaneously, or could close, when said needle is removed.

Also the perforable closing device associated to closed venous catheter systems and the like, they must provide the sealing either when they are engaged by the needle or when the latter is removed. For this purpose they consist of elastomer material elements located in a seat along the housing of the needle made in a body means, usually made of plastic, connecting the cannula and the pipes or the fittings for the pipes for the liquids to be injected or to be tapped from a biological body.

The known perforable closing devices, in particularly if they are very old and stored in severe environment conditions, can cause unwanted and dangerous leaks of injectable liquid or of blood, more often in the final phase of removing of the needle and also after the needle removing.

Furthermore the force needed to perforate the device and to remove the needle from the device can be too intense provoking risks for the patient and for the medical operator, particularly in case of devices associated to cannula or to catheters.

In order to overcome said drawbacks, the document US6506181 provides the use of a device made of two cup shaped bodies kept in mutual contact with the respective mouths mutually facing or mutually fixed to form an inner cavity.

This known solution improves the sealing and the flow of the needle but it is affected by the drawback to be made of a plurality of parts that must be perfectly assembled for assuring the needed operation; a further drawback consists of the excessive cost of the parts and of the assembly thereof.

Other drawback consists in that said known solution seems not appropriate to the production of closures for bottles and for test tube, and for injections tips for injection pipes and for dialysis.

Also the prior documents N. WO 2007/044878, N. WO 2007/050788, N. WO 98/30276 and N. US 6 352 520 refers to medical devices of kind needle - cannula or to components thereof provided with a sealing that, particularly if stored in severe environment conditions, could not ensure the sealing after a limited period of storage.

An object of the present invention is to propose a sealing perforable closing device for a perfect sealing before and after the removal of the needle also after a along period of time of storage in very severe environment conditions, for instance at very high temperatures.

A further object is to propose a device for a reduced sliding friction of the needle also when it is used close to the void date of the product and also after many years from production.

Further object is to propose a device suitable for the closed venous catheter systems and the like and also suitable for the perforable closures of bottle, perforable injections tips for infusion or for dialysis devices and the like.

Other object is to propose an inexpensive device, easy to be assembled.

Further object of the present invention is to propose a medical apparatus associated to said sealing perforable closing device.

The features of the invention are highlighted with particular reference to the enclosed drawings in which:
- figure 1 shows a schematic and partially sectioned view of a medical apparatus object of the present invention associated to the sealing perforable closing device object of the present invention, in a condition of partial removal of the needle from the venous catheter device;
- figure 2 shows the apparatus and the device of figure 1 in a complete removal condition of the needle;
- figure 3 shows an enlarged view of a particular of figure 1;
- figure 4 shows an axonometric projection and enlarged view of a first embodiment of the sealing perforable closing device of figure 1;
- figure 5 shows an axonometric projection and enlarged view of a second embodiment of the sealing perforable closing device of figure 1;
- figure 6 shows a side view of the device of figure 5;
- figure 7 shows a cross sectional view according to the plane VII - VII of figure 6;
- figure 8 shows a side view of a third embodiment of the device of figure 1;
- figure 9 shows a cross section view according to the plane IX - IX of figure 8;
- figures 10 and 11 show respectively axial and side views of a fourth embodiment of the device of figure 1;
- figure 12 shows a cross section view according to the plane XII - XII of figure 11;
- figure 13 shows a side view of a fifth embodiment of the device of figure 1;
- figure 14 shows a cross section view according to the plane XIV - XIV of figure 13;
- figure 15 shows a side view of a sixth embodiment of the device of figure 1;
- figure 16 shows a cross section view according to the plane XVI - XVI of figure 15;
- figures 17 and 18 show side views of respective further embodiments seventh and eighth of the device of figure 1.

With reference to the figures, in the following are described several embodiments of the invention bound by the same inventive concept and reported from the simplest to the most complex 1.

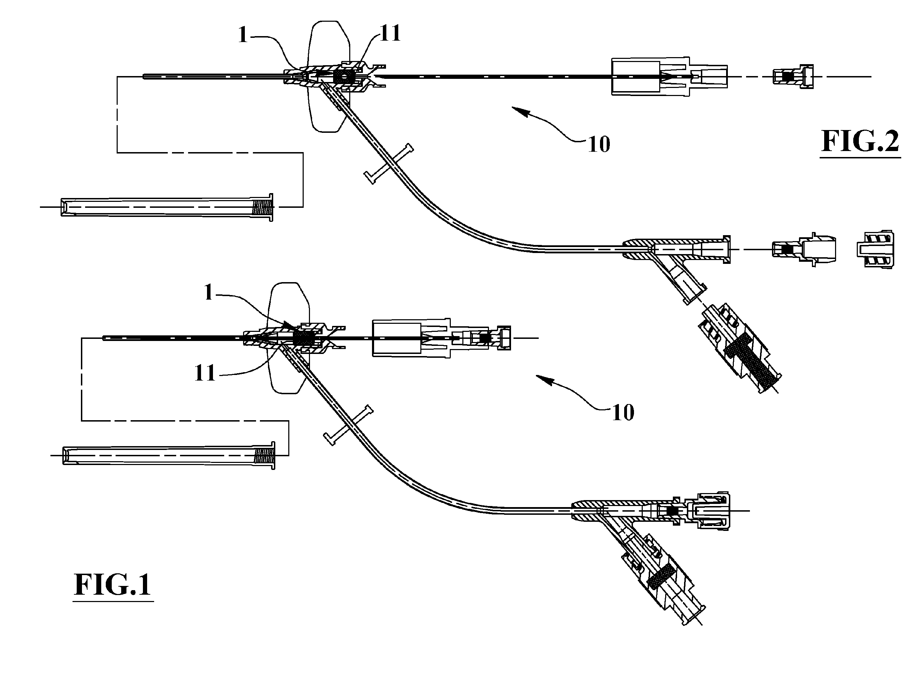

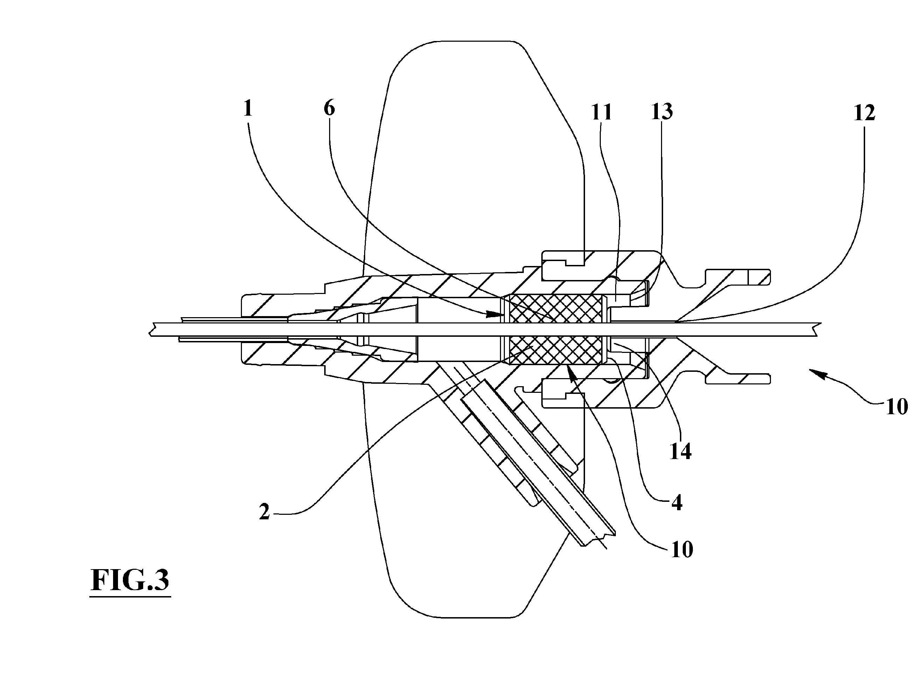

Referring to figures 1 - 4, numeral 1 indicates the sealing perforable closing device and numeral 10 indicates a medical apparatus provided with a housing 11 for said device 1 object of the present invention. Said housing 11 is made of stiff material and it houses the device.

Housing 11 and device 1 have almost the same transversal sections so the side face of the device contacts the inner side wall of the housing 11.

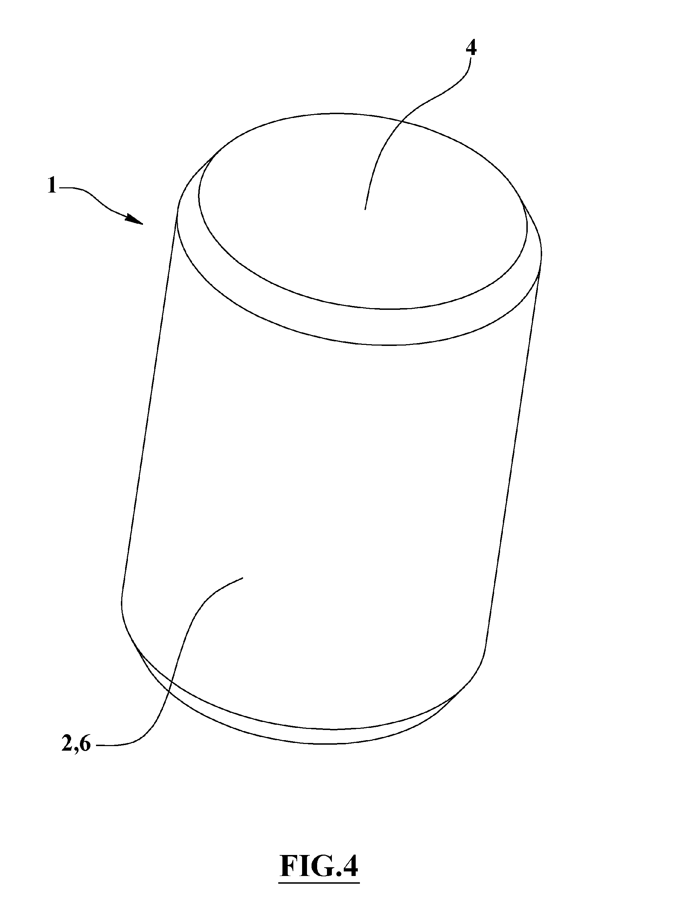

Figure 4 shows the device 1 detached from the medical apparatus.

The sealing perforable closing device 1 is seated into the respective housing 11 of the medical apparatus and in the initial operative conditions is engaged by a detachable injection needle perforating it along a piercing direction. Said device 1 comprises an elongated shaped body means 2, preferably made integral in a single body.

The body means 2, when is seated into the housing 11 and is crossed by the needle that perforates it, occupies almost all the transversal section of said housing 11.

The body means 2 is preferably complementary shaped in respect to the housing 11 or to a portion thereof and it can be prismatic, frustoconical or preferably cylindrical shaped and its geometric longitudinal axis is aligned to the piercing direction when the body means 2 is seated into the respective housing 11. Said body means 2 is made of elastomer material and comprises an absorbent material 6 fit for absorbing liquids.

The absorbent material 6 can be mixed, dispersed, englobed or adsorbed into the elastomer material.

The absorbent material 6 can consist, for instance, in one or more polymers or copolymers pure or mixed; in particular the absorbent material 6 can be based on Polyacrylic Acid Sodium CAS 9003-04-7.
In alternative the absorbent material 6 can consist or comprise by products of or from the silicon or material of vegetable origin, for instance cellulose by products.

The elastomer material of the mix or dispersion can be, for instance, of a kind known in medical applications.

Optionally, and particularly in some applications in which it is possible that the liquid contacts the housing 11 before the removal of the needle, the device 1 can comprise a diaphragm element, not shown, for instance in the shape of a disc made of water-proof elastomer material. Said diaphragm element is positioned into the end of the housing 11 that can be flood by the liquid at least for preventing reflux of liquids.

The working of the device 1 in an operative condition in which is seated into its housing 11 of the medical apparatus 10, it provides that, during removal and detaching of the needle or in other conditions, the device 1 and in particular the perforation without the needle, it can contact medical liquids, blood or liquids from the body in general. In such a case, the absorbent material 6, mixed, dispersed, incorporated or adsorbed into the elastomer material, it absorbs the liquid.

If the quantity of said liquid is enough, the liquid causes an increasing of volume of the body means 2. Said increasing of volume, due to the housing of the device against the inner side wall of the stiff housing 11, causes the compression and the occlusion of the perforation and of any other passage through the device 1 or through the housing 11.

In other words, an amount of liquid above a certain threshold contacting the device causes the expansion of the device 1 so the latter 1 occupies also the volume of the removed needle and of any possible gap between the device and the side wall of the housing 11.

Small quantity of liquids, if not enough to cause the expansion needed for the closure, are immediately absorbed by the body means 2 of the device 1 that renders harmless said liquids; possible further leaks of liquids, reaching the device 1, can cause the expansion of the body means and the occlusion of the perforation.

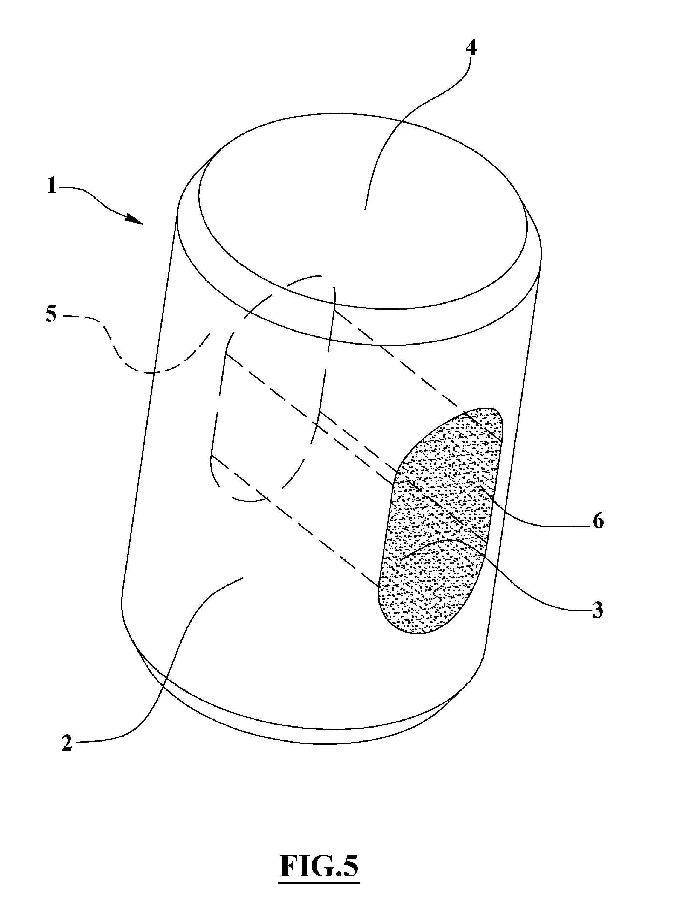

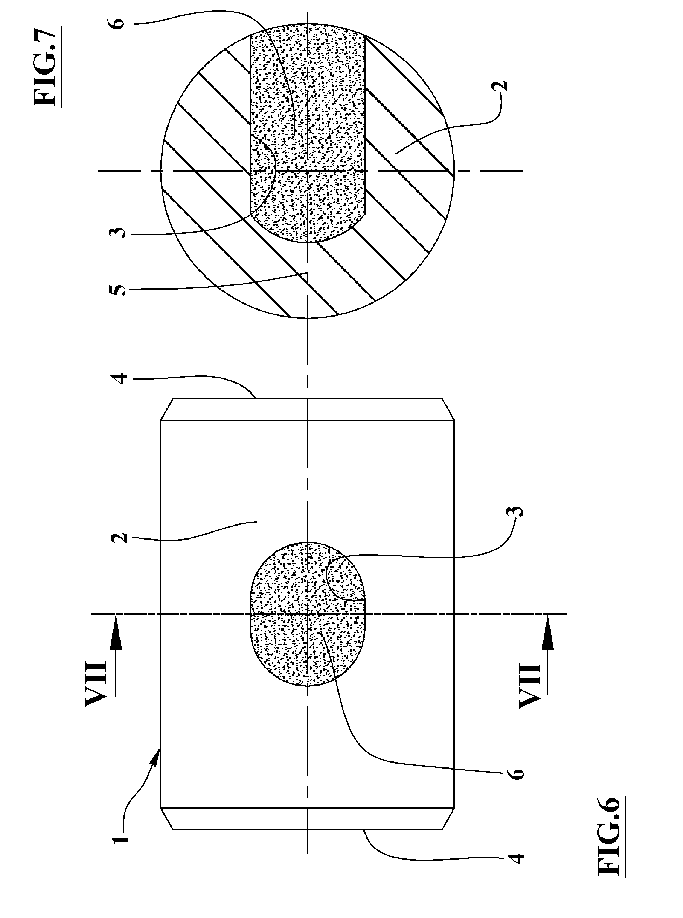

The second embodiment of figures 5 - 7 differs from the preceding embodiment due to the fact that the absorbent material 6 is housed in a concave seat 3 carried out into the body means 2 of the device 1 and said body means is made of elastomer material, for instance of the kind known in the medical application.

The seat 3 is transversally oriented in respect to the piercing direction, in other words, it 3 is oriented transversally in respect to the geometric longitudinal axis of the body means 2, and said seat 3 crosses said piercing direction so that the needle crosses said seat 3 and the absorbent material 6 housed inside the seat.

One end of the seat 3 is opened and it flows outside of the body means 2, the opposed end is closed by a diaphragm 5, or by a septum or the like of the body means 2.

Said diaphragm 5 or the like is integral with the body means 2.

The geometric longitudinal axis of the seat 3 is straight and perpendicular to the longitudinal axis of the body means 2, said longitudinal axis of the seat 3 crosses the longitudinal axis of the body means 2 in a point of mutual incidence.

The transversal sections of the seat 3 are elliptical, oval shaped or the like having the main axis parallel to the longitudinal axis of the body means 2.

In alternative the invention provides that the transversal sections of the seat 3 are round, rectangular, squared, triangular, or other geometric regular or irregular shapes.

The bases 4 of the cylindrical body means 2 are orthogonal in respect to the geometric longitudinal axis of the body means.

The absorbent material 6 of liquids, for instance comprising or consisting of Polyacrylic Acid Sodium CAS 9003-04-7, is in form of powder, pellets, micro pellets, granules, or grains in tablets complementary shaped in respect to the seat 3 or holed complementary tablet.

Clearly the absorbent material 6, in particular in form of not holed tablet, must be not excessively solid or hard for not hamper the insertion and/or removal of the needle; vice versa the needle slides easily because of the low coefficient of friction between the needle and the absorbent material 6 or because of the hole.

In alternative the invention provides that the absorbent material 6 contained in each seat 3 is in form of pad made of an elastomer product charged with said absorbent material 6. In other words, the invention provides that in the present and in the following embodiments, each seat 3 can contain a pad, filling all or almost all the volume of the seat 3; said pad is made by a matrix of an elastomer product, equal or different from the elastomer material of the body means 2; said matrix is charged with solid particles or powder of the absorbent material 6.

The volume percentage of the absorbent material 6 into the pad can range between 2% and 97%, preferably between 25% and 35%. Said technical solution prevents the tip of the needle to carry free particles of absorbent material during the production phase of inserting the needle inside the device and the apparatus.

The first embodiment of figures 1 - 4 can be seen as the pad releasing the full body means 2.

In the present second embodiment, the expansion of the body means 2 is due to the increasing of the volume of the seat 3 following the contact of the absorbent material with a liquid.

In this embodiment the extraction force for removing the needle is reduced because of the lower total friction of sliding the needle through the device 1.

The device 1 can be associated to the medical apparatus shown in figures 1 - 3 and in general to venous catheter systems or to a bottles closure provided with a housing for the device.

Furthermore, the device 1 can be associated to an injections tips for fluid administration devices or for dialysis and to a sample test tubes and to the like.

In the first case, the operation provides that after the insertion of the cannula into a segment of the blood vessel, the needle is removed sliding it in the piercing direction along the body means 2 of device 1.

The features of the body means, and in particular of the seat 3 containing the absorbent material, make easier the sliding of the needle preventing painful and dangerous jerks that, through the needle the same and/or the cannula, could reach the body of the patient.

Furthermore, the surface of the needle, during sliding towards the removed condition, meets in succession the surface of the first base 4 of the body means and the surface of the inner wall of the seat 3; both said surfaces can detach blood or other liquid from the needle and from its tip helping the absorbing of said liquids by the absorbent material 6.

Possible remaining liquids penetrating into the seat 3 are absorbed by the absorbent material 6 and can't follow the surface of the needle and its tip, so said remaining liquids are blocked.

When the absorbent material 6 absorbs a preset quantity of liquid, it expands itself closing the perforation and sealing possible outflows of medical liquids or body liquids.

The features of the body means, and in particular its special seat, increase the elastic deformability of the body means during the removal of the needle improving the sealing capability of said body means also after many years or in severe environment conditions.

When the device is seated in a respective housing of a bottle closure of the kind for injections or of dialysis tips of of test tubes, it is provided that the device is pierced by the needle at use only. Also in this case, the features of the device facilitates the perforation, preventing the removed needle is contaminated by the liquid of the bottle, of the tube or of the injection or dialysis; said features furthermore improve the sealing of the device 1 after its use.

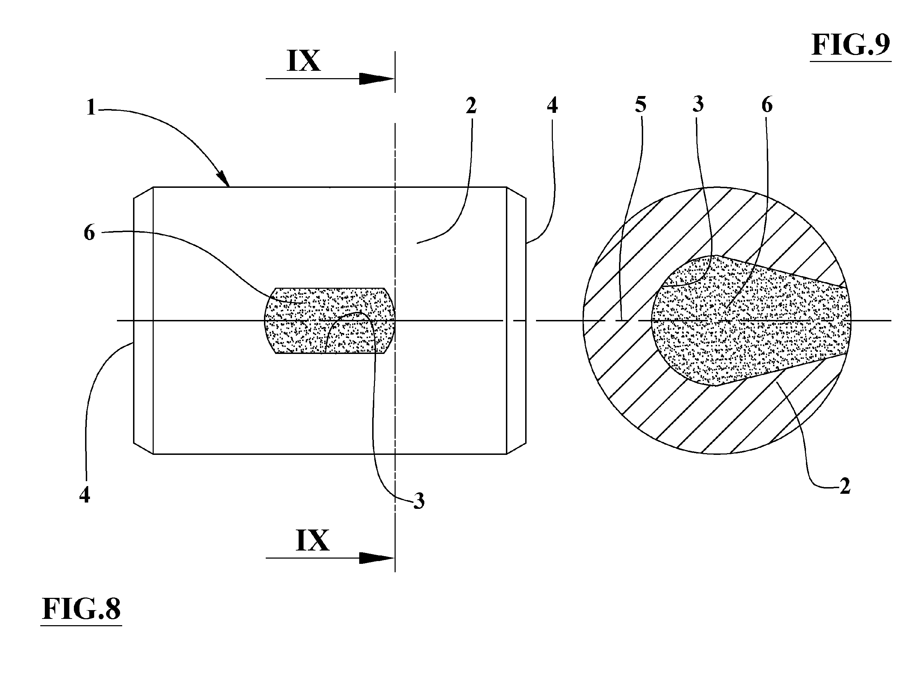

The third embodiment of figures 8 and 9 differs from the preceding embodiment in that the transversal sections of the seat 3 decrease towards its opened end.

Said seat is shaped approximately as a pear, a truncated cone, a matrass or the like. Said embodiment allows to house a bigger quantity of absorbent material without making thinner and without excessively weakening the body means 2.

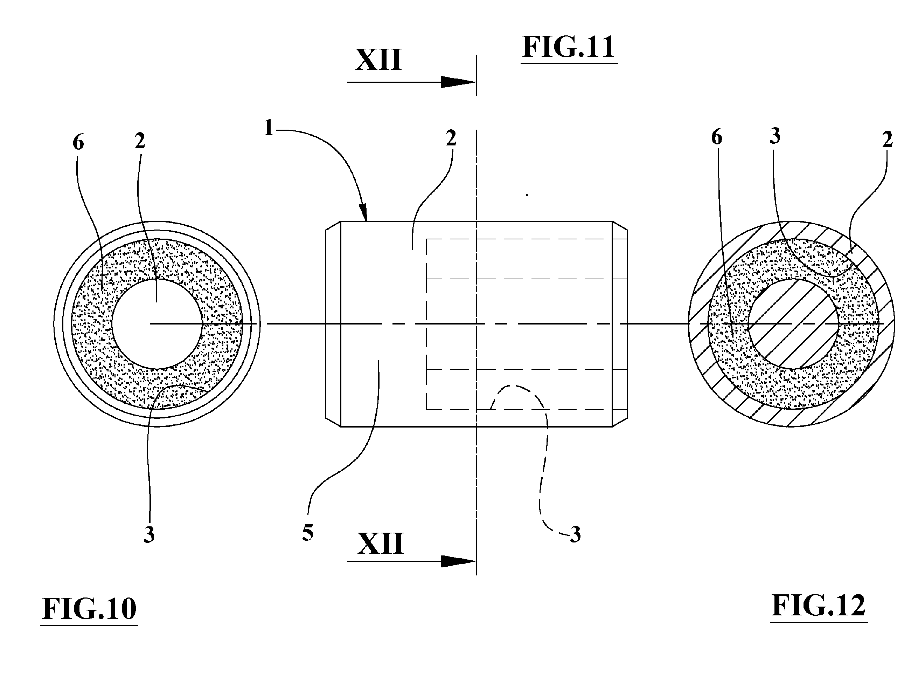

The fourth embodiment of figures 10 - 11 differs from the second embodiment in that the seat 3 is cylindrical wall shaped with respective geometrical axis almost coinciding with the geometric longitudinal axis of the body means 2.

The transversal sections of the seat 3 are annulus shaped.

An end of the cylindrical wall seat 3 outflows at an end of the body means 2; the opposed end of the seat 3 is closed by a septum 5.

In this embodiment, the needle crosses a bigger number of interfaces between absorbent material - elastomer material of the body means providing a better cleaning of the removed needle.

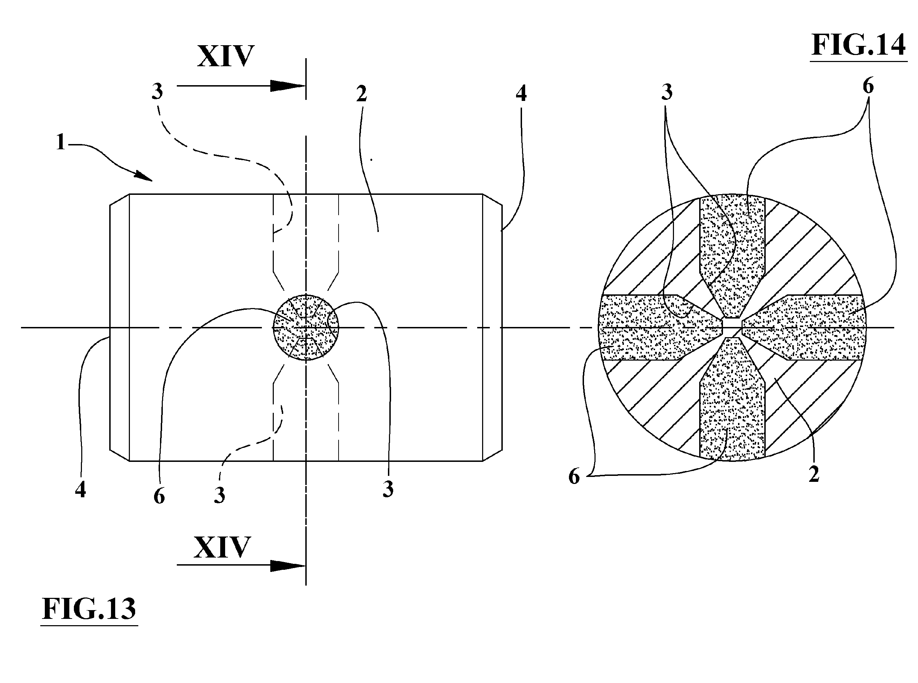

The invention includes the possibility that the body means is provided with two or more seats 3 and in particular the fifth embodiment of figures 13 and 14 provides that the body means 2 is equipped with four separated seats 3, converging towards a point of the longitudinal axis of the body means 2. Said seats 3 are almost angularly regularly spaced apart and they lay on a transversal geometrical plane of the body means 2.

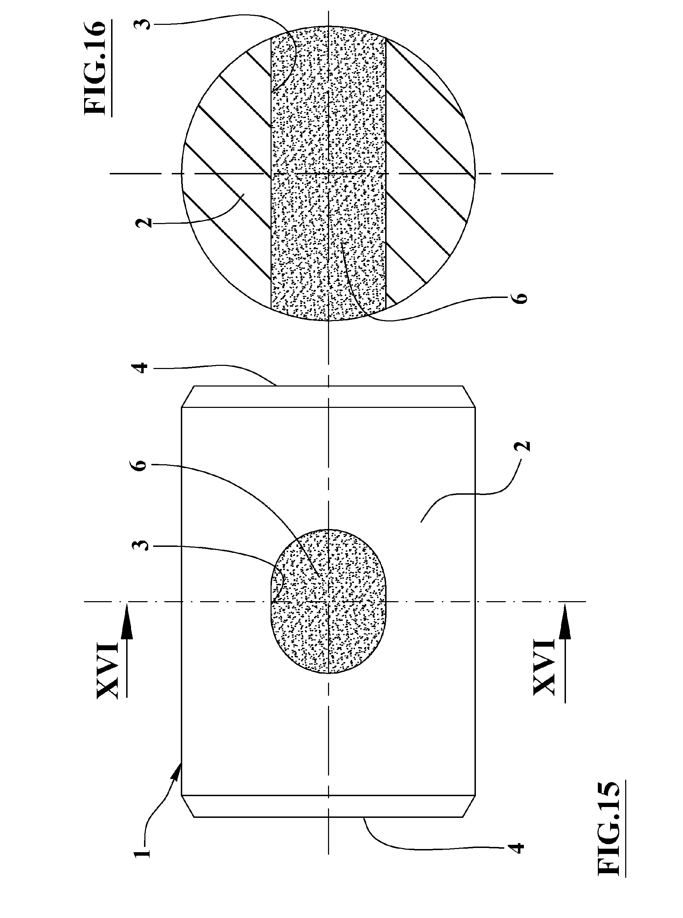

The sixth embodiment of figures 15 and 16 differs from the second embodiment of figure 5 in that both the ends of the seat 3, containing the absorbent material, are opened and flow outside of the body means.

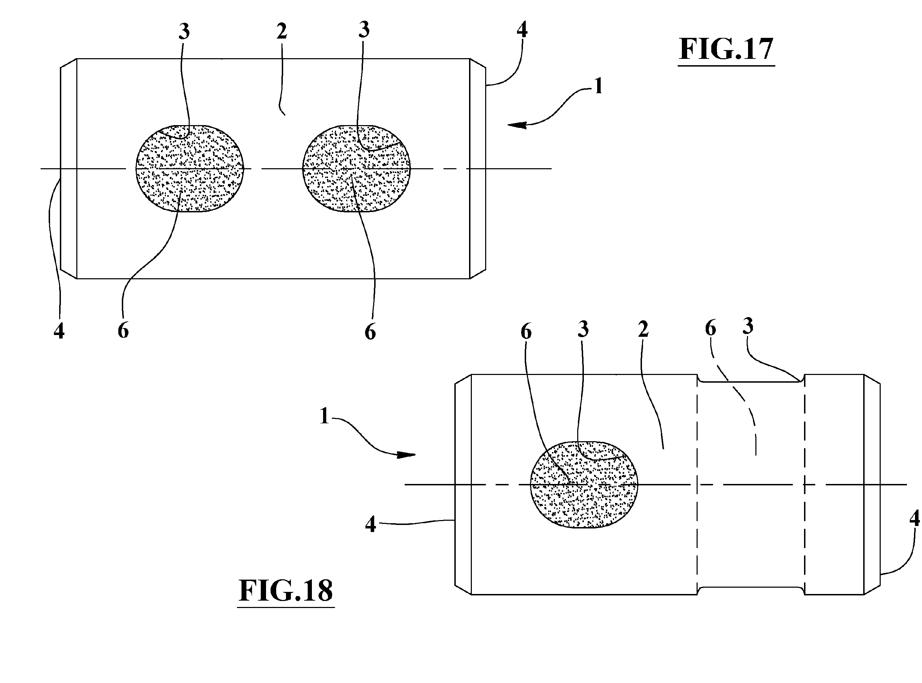

The seventh embodiment of figure 17 differs from the preceding embodiment of figures 15 and 16 in that the body means 2 is provided with two seats 3 whose longitudinal axis are mutually parallel. This latter embodiment increases the total volume of absorbent material, further reduces the force for sliding of the needle and improve the capability of removing the fluids from the external surface and from the tip of the needle.

The eighth embodiment of figure 18 differs from the preceding embodiment of figure 17 in that the longitudinal axis of the two seats 3 are mutually inclined at about 90°.

Said embodiment provides the similar advantage of that of figure 8 but is particularly suitable when the possibility to cross at least one of the two cavities must be increased especially when the point of insertion of the needle is offset.

In order to reduce the costs and/or to facilitate the production of the two latter embodiments, the respective body means can be produced in two parts, each of them being provided with a respective seat.

The invention provides that the volume of the absorbent material 6 of the body means 2 of each embodiment is equal to or preferably bigger than the minimum quantity necessary to obtain the expansion of the body means and the occlusion of the perforation in case of contact with a sufficient quantity of liquids. In such a manner, the possibility of liquids flow through the housing 11 is prevented.

In case of big quantity of liquids and of big increase of volume of the body means 2, the latter 2 can expand itself longitudinally, occupying the free volumes of the housing avoiding excessive increasing of pressure and risks of damaging of the medical apparatus.

The medical apparatus object of the present invention is associable to the device 1 of any of the preceding embodiments and it can release a closed venous catheter systems and the like or the perforable closures of intravenous drip bottle closure, bags closure, perforable injections tips for infusion or dialysis devices and the like.

The apparatus 10 comprises a housing for the device 1 and a housing for the needle 12 with respective longitudinal geometric axis coinciding with a piercing direction.

The housing 11 is provided with stiffen material walls which are able to support the pressure exerted by the body means when the absorbent material 6 contacts a liquid.

The inner volume limited by the housing 11 is prismatic, frustoconical or preferably cylindrical shaped.

When in use, the needle of the apparatus is engaged into the device 1, the needle 12 being inserted inside the body means and through the device during production, and it 12 contacts the absorbent material 6.

The side wall of the housing 11 for the device 1 is assigned to seal match the side surface of the body means 2 of the device 1.

In case of intravenous system, the needle provides a guide for a flexible cannula allowing the insertion thereof into a portion of the blood vessel.

After the introduction of the cannula into the vessel, the needle must be removed and it slides, in said piercing direction, along the device 1. The latter 1, being made of elastomer material and containing the absorbent material 6, absorbs possible liquid contacting it, it expands and it closes the hole of the perforation sealing the outlet of the needle.

The end 13 of said housing for the device 1 that meets last the needle during the removal thereof, in other words, the end 13 opposed to the cannula, is provided with a cylindrical protrusion 14 that is coaxial in respect to the housing 11 and having a diameter smaller in respect to the diameter of the body means 2 of the device 1. Said cylindrical protrusion 14 matches a base 4 of the body means 2 and unexpectedly improves the cleaning of the removed needle and it improves the sealing of the apparatus; furthermore it facilitates the insertion into the housing blocking the body means 2.

The housing can be equipped with annular protrusion or ribs fit for matching the end of the device opposed in respect to the end matching the cylindrical protrusion 14 for blocking or for confining the longitudinal motion of the body means 2.

An advantage of the present invention is to provide a sealing perforable closing device for a perfect sealing before and after the removal of the needle also after a along period of time of storage in very severe environment conditions, for instance at very high temperatures.

A further advantage is to provide a device for a reduced sliding friction of the needle also when it is used close to the void date of the product and also after many years from production.

Further advantage is to provide a device suitable for the closed venous catheter systems and the like and also suitable for the perforable closures of intravenous drip bottle closure, bottle, perforable injections tips for infusion or dialysis devices and the like. Other advantage is to provide an inexpensive device, easy to be assembled.

Further advantage of the present invention is to provide a medical apparatus associated to said sealing perforable closing device.

## Claims

1. Sealing perforable closing device associable to a medical apparatus provided with a housing for said device (1) and engageable by a needle along a piercing direction; said device (1) being **characterized in that** it comprises a body means (2) including an absorbent material (6) for absorbing liquids; in an operative condition of extraction of the needle, possible liquids contacting the absorbent material are absorbed and said absorbed liquids can cause an increasing of volume of the absorbent material and of the body means (2), the latter (2) expands itself and seals the perforation that passes through the body means (2).

2. Device according to claim 1 **characterized in that** the body means is prismatic, frustoconical or cylindrical and its geometric longitudinal axis is aligned to the piercing direction when the body means (2) is seated into the housing; preferably the body means (2) is cylindrical ant its bases (4) are orthogonal to its geometric longitudinal axis.

3. Device according to claim 1 or the 2 **characterized in that** the body means (2) is provided with at least a concave seat (3) interfering with the piercing direction when the body means (2) is seated into the housing, said seat (3) contains the absorbent material (6) of the body means (2).

4. Device according to claim 3 **characterized in that** the at least a seat (3) of the body means (2) is transversally oriented in respect to the piercing direction.

5. Device according to claim 3 or 4 **characterized in that** an end of the at least a seat (3) is opened and flows outside the body means; the other end of the at least a seat (3) is closed by a diaphragm (5), septum or the like.

6. Device according to any of the claims 3 - 5 **characterized in that** the transversal sections of the at least a seat (3) are elliptical, oval or the like, the main geometric axis of said sections are parallel to the longitudinal axis of the body means (2); or said transversal sections are round, squared, triangular, rectangular or the like.

7. Device according to any of the claims 3 - 6 **characterized in that** the transversal sections of the at least a seat (3) decrease towards its opened end and said seat is shaped approximately as a pear, a truncated cone, a matrass or the like.

8. Device according to claim 3 or 5 **characterized in that** the seat (3) is cylindrical wall shaped where the geometric axis of the cylindrical wall almost coincides with the longitudinal axis of the body means (2), the cross sections of the seat (3) being annulus shaped.

9. Device according to any of the claims 1 - 4 or 6 - 8 **characterized in that** both the ends of each seat (3) are opened and flow outside of the body means and/or the body means (2) is provided with at least two seats (3); preferably the seats could be in number of two and they could have all the ends opened, said seats have mutually parallel or inclined longitudinal axis.

10. Device according to claim 6 **characterized in that** the body means (2) is provided with a plurality of seats (3), preferably four, converging towards a point of the longitudinal axis of the body means (2), said seats (3) are almost angularly regularly spaced apart and they lay on a transversal geometrical plane of the body means (2).

11. Device according to any of the preceding claims **characterized in that** the absorbent material (6) consists in one or more polymers or copolymers pure or mixed; the absorbent material (6) can contain or can be based on Polyacrylic Acid Sodium CAS 9003-04-7 and/or the absorbent material (6) can consist of, or comprise, by products of the silicon or material of vegetable origin such as cellulose by products.

12. Device according to any of the preceding claims **characterized in that** the absorbent material (6) is in form of powder, pellets, micro pellets, granules, or grains in tablets.

13. Device according to any of the claims 1 - 11 **characterized in that** said absorbent material (6) contained in each seat (3) is in form of a pad comprising an elastomer product charged with said absorbent material (6).

14. Device according to claim 2 **characterized in that** the absorbent material (6) is dispersed in, or mixed to an elastomer material, said absorbent and elastomer materials together release the body means (2).

15. Medical apparatus associable to the device (1) of any of the preceding claims; said apparatus (10) being **characterized in that** it comprises a stiff housing (11) for the device (1) and a housing for the needle (12) whose respective longitudinal geometric axis coincide with a piercing direction where the side wall of the housing for the device (1) is assigned to sealing match a side wall of a body means (2) of the device (1); the end (13), in the direction of the removal motion of the needle, of said housing (11) for the device (1) is provided with a cylindrical protrusion (14) that is coaxial in respect to the latter housing (11) and having a smaller diameter than the body means (2) of the device (1); said cylindrical protrusion (14) being assigned to match the base (4) of the body means (2).
